(19) **Europäisches Patentamt / European Patent Office / Office européen des brevets**

(11) **EP 4 343 307 B1**

(12) # EUROPEAN PATENT SPECIFICATION

(45) Date of publication and mention
of the grant of the patent:
**13.05.2026 Bulletin 2026/20**

(21) Application number: **22827296.9**

(22) Date of filing: **25.05.2022**

(51) International Patent Classification (IPC):
**G01N 21/25** *(2006.01)*    **G01N 21/78** *(2006.01)*
**G01N 31/22** *(2006.01)*    G01N 21/77 *(2006.01)*

(52) Cooperative Patent Classification (CPC):
**G01N 21/251; G01N 21/78; G01N 31/22;**
G01N 2021/7763; G01N 2201/121;
G01N 2201/1296

(86) International application number:
**PCT/CN2022/094832**

(87) International publication number:
**WO 2022/267799 (29.12.2022 Gazette 2022/52)**

(54) **WATER QUALITY TESTING METHOD AND WATER QUALITY TESTING APPARATUS**

WASSERQUALITÄTTESTVERFAHREN UND WASSERQUALITÄTTESTVORRICHTUNG

PROCÉDÉ D'ESSAI DE LA QUALITÉ DE L'EAU ET APPAREIL D'ESSAI DE LA QUALITÉ DE L'EAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.06.2021 CN 202110695994
23.06.2021 CN 202110696024**

(43) Date of publication of application:
**27.03.2024 Bulletin 2024/13**

(73) Proprietors:
• **China Petroleum & Chemical Corporation
Beijing 100728 (CN)**
• **Sinopec Research Institute of Safety
Engineering Co., Ltd.
Qingdao, Shandong 266104 (CN)**

(72) Inventors:
• **FENG, Junjie
Qingdao, Shandong 266104 (CN)**
• **SUN, Bing
Qingdao, Shandong 266104 (CN)**
• **JIANG, Huiyun
Qingdao, Shandong 266104 (CN)**
• **JIN, Yan
Qingdao, Shandong 266104 (CN)**
• **XIAO, Anshan
Qingdao, Shandong 266104 (CN)**

• **WANG, Haozhi
Qingdao, Shandong 266104 (CN)**
• **WANG, Shiqiang
Qingdao, Shandong 266104 (CN)**

(74) Representative: **karo IP
Patentanwälte PartG mbB
Steinstraße 16-18
40212 Düsseldorf (DE)**

(56) References cited:
WO-A1-2018/095412    CN-A- 106 770 224
CN-A- 107 144 531    CN-A- 107 240 089
CN-A- 109 859 117    CN-A- 109 859 117
CN-A- 110 579 471    RU-C1- 2 682 162

• **CARVALHO PEDRO H ET AL: "Estimation of
Sulfonamides Concentration in Water Based on
Digital Colourimetry", 22 September 2019,
PATTERN RECOGNITION AND IMAGE
ANALYSIS; [LECTURE NOTES IN COMPUTER
SCIENCE; LECT.NOTES COMPUTER],
SPRINGER INTERNATIONAL PUBLISHING,
CHAM, PAGE(S) 355 - 366, ISBN:
978-3-030-31331-9, ISSN: 0302-9743,
XP047670521**

- NURIA LOPEZ-RUIZ ET AL: "Smartphone-Based Simultaneous pH and Nitrite Colorimetric Determination for Paper Microfluidic Devices", ANALYTICAL CHEMISTRY, vol. 86, no. 19, 7 October 2014 (2014-10-07), US, pages 9554 - 9562, XP055241637, ISSN: 0003-2700, DOI: 10.1021/ac5019205
- COSTA DIOGO ET AL: "The Nutrient App: Developing a smartphone application for on-site instantaneous community-based NO3 and PO4 monitoring", ENVIRONMENTAL MODELLING & SOFTWARE, ELSEVIER, AMSTERDAM, NL, vol. 133, 29 August 2020 (2020-08-29), XP086284567, ISSN: 1364-8152, [retrieved on 20200829], DOI: 10.1016/J.ENVSOFT.2020.104829
- CAO PINGPING ET AL: "Chromaticity Measurement Based on the Image Method and Its Application in Water Quality Detection", WATER, vol. 11, no. 11, 8 November 2019 (2019-11-08), CH, pages 2339, XP093201442, ISSN: 2073-4441, DOI: 10.3390/w11112339

**Description**

**Field of the Invention**

The present disclosure relates to the technical field of environmental monitoring, in particular, to a water quality testing method and a water quality testing device.

**Background of the Invention**

The quantitative measurement of a target substance in a body of water is widely used in processes such as industrial water quality testing, environmental testing, biochemical analysis, accident investigation, domestic water testing, sewage treatment, and relates to the fields of industrial production, medical health, and daily life. Water quality testing techniques mainly include chemical analysis methods and instrumental analysis methods and the like, such as gravimetric analysis, titration analysis, optical analysis, electrochemical analysis, chromatography, mass spectrometry and the like. Many of the conventional testing methods have a large apparatus volume, a slow analysis speed, low work efficiency and a large amount of reagents, which are difficult to adapt to the on-site and field working environment conditions and difficult for portable use. While the development of large and sophisticated monitoring systems continues, there is a growing interest in the development of small, portable, self-continuous, simple and rapid monitoring techniques. To address the problems of inefficient testing and harsh monitoring conditions of existing water body target substance quantitative determination methods, there is a need to create a water quality testing method based on digital image processing according to a small portable testing apparatus. Carvalho, Pedro H., et al. "Estimation of sulfonamides concentration in water based on digital colourimetry" in: Iberian Conference on Pattern Recognition and Image Analysis. Cham: Springer International Publishing, 2019. S. 355-366, describes a method of determining the concentration of sulfonamides concentration in water. In the proposed method, photographs of samples next to a colour reference target are acquired to build a dataset. An algorithm is used to detect the reference target, based on binarisation algorithms, in order to standardise the collected images using a colour correction matrix converting from RGB to XYZ, providing a necessary colour constancy between photographs from different devices. Afterwards, the sample is extracted through edge detection and Hough transform algorithms. Finally, the sulfonamide concentration is estimated resorting to an experimentally designed calibration curve, which correlates the concentration and colour information.

Lopez-Ruiz Nuria, et al. "Smartphone-based simultaneous pH and nitrite colorimetric determination for paper microfluidic devices" in Analytical chemistry, 2014, 86. Jg., Nr. 19, S. 9554-9562, describes an Android application for measurement of nitrite concentration and pH determination in combination with a low-cost paper-based microfluidic device. The application uses seven sensing areas, containing the corresponding immobilized reagents, to produce selective color changes when a sample solution is placed in the sampling area, and a reference area used to normalise the color coordinates of the sensing areas. Under controlled conditions of light, using the flash of the smartphone as a light source, the image captured with the built-in camera is processed using a customized algorithm for multidetection of the colored sensing areas.

**Summary of the Invention**

The present disclosure provides a water quality testing method and a water quality testing device aiming at the technical problems in the prior art that a complex testing apparatus is required, the testing application range is narrow and the testing environment is high. The method is simple, the testing speed is fast, the application range is wide, the method does not depend on specific photographing conditions, the method has good applicability to different photographing apparatuses, light sources and photographing methods, and the testing accuracy is high.

In order to achieve the above object, the present invention provides a water quality testing method according to claim 1, the method comprising: obtaining a test sample, the test sample comprising a target test substance;

    determining a reference substance corresponding to the test sample;
    determining a third chromaticity of the reference substance under a first environment, and a fourth chromaticity of the reference substance under a second environment;
    obtaining a chromaticity-concentration mapping relationship characterizing a mapping relationship between the chromaticity of the test sample under the second environment and the concentration of the target test substance;
    determining an absolute value of the chromaticity difference between the fourth chromaticity and the third chromaticity, and

-    if an absolute value of the chromaticity difference between the fourth chromaticity and the third chromaticity is greater than a preset deviation threshold:

constructing a chromaticity correction model by a neural network algorithm or a multivariate non-linear fitting method according to a chromaticity difference between the fourth chromaticity and the third chromaticity, acquiring a first chromaticity of the test sample under the first environment, determining a second chromaticity of the test sample under the second environment based on the first chromaticity and the chromaticity correction model, and

determining a concentration of the target test substance based on the determined second chromaticity and the chromaticity-concentration mapping relationship; and

- in a case where the absolute value of the chromaticity difference between the fourth chromaticity and the third chromaticity is less than or equal to the preset deviation threshold, the method further comprises:

acquiring the chromaticity of the test sample under the first environment, and
obtaining the concentration of the target test substance in the test sample based on the chromaticity-concentration mapping relationship and the chromaticity of the test sample under the first environment;
wherein the first environment is a test site environment in which the test sample is located; and the second environment is a laboratory standard environment.

According to an embodiment, obtaining the test sample includes: judging whether an aqueous solution of the target test substance is colored or not, and judging whether the aqueous solution of the target test substance has an interference color or not; taking the aqueous solution of the target test substance as a test sample if the aqueous solution of the target test substance is colored and has no interference color; selecting a corresponding test reagent according to the target test substance if the aqueous solution of the target test substance is colorless or has an interference color, and fusing the selected test reagent with the aqueous solution of the target test substance to obtain a test sample.

According to an embodiment, the test reagent corresponding to the target test substance is a test reagent that undergoes a chromogenic reaction upon fusion with an aqueous solution of the target test substance.

According to an embodiment, determining the third chromaticity of the reference substance under the first environment, and the fourth chromaticity of the reference substance under the second environment includes: acquiring color image information of the reference substance under the second environment, and performing color image information processing according to a preset image algorithm; performing chromaticity extraction on the processed color image information of the reference substance under the second environment to obtain a fourth chromaticity of the reference substance under the second environment; acquiring color image information of the reference substance under a first environment, and performing color image information processing according to a preset image algorithm; performing chromaticity extraction on the processed color image information of the reference substance under the first environment to obtain a third chromaticity of the reference substance under the first environment.

According to an embodiment, the preset image algorithm includes at least: image transformation, key region selection, edge testing, noise reduction, smoothing and chromaticity enhancement.

According to an embodiment, the color image information chromaticity extraction under the second environment and the color image information chromaticity extraction under the first environment are performed under a same chromaticity system, and the chromaticity system is any one of: RGB, HSV, CMYK, CIE, and LAB.

According to an embodiment, before performing color image information processing on the color image information of the reference substance according to the preset image algorithm, the method further includes: determining that the color image information of the reference substance meets a testing requirement, and determining that the color image information of the test sample meets the testing requirement.

According to an embodiment, the testing requirement includes at least: image pixels, illumination intensity and light uniformity of a color image.

According to an embodiment, the method further includes: constructing the chromaticity-concentration mapping relationship including: selecting a plurality of aqueous solutions containing the target test substance, wherein the concentration of the target test substance in each aqueous solution is known and different; acquiring chromaticities of the plurality of aqueous solutions including the target test substance under the second environment; obtaining a function relationship between the concentration of the target test substance and the chromaticity of the aqueous solution of the target test substance under the second environment based on the concentration of the target test substance in each aqueous solution and the corresponding chromaticity of each aqueous solution under the second environment, and obtaining the chromaticity-concentration mapping relationship from the function relationship.

According to an embodiment, the reference substance is a standard color card; a plurality of color regions exist on the standard color card, when performing reference substance color degree extraction, chromaticities of all the color regions on the standard color card are simultaneously extracted to obtain a first chromaticity set; the first chromaticity set is subjected to chromaticity correction model train as a training sample.

According to an embodiment, the reference substance is a paper microfluidic chip, a color card, a microchannel chip or a color developing apparatus; the reference substance is colored by any one of the following modes: an intrinsic color of the reference substance, dye addition, addition of a color reaction system.

According to an embodiment, the chromaticity correction model construction is performed using a neural network algorithm and a multivariate non-linear fitting method respectively, and the two constructed chromaticity correction models are trained respectively based on a certain selected reference substance, and training results of the two chromaticity correction models are obtained; the training results of the two chromaticity correction models are compared: when an absolute value of a difference between the two is less than a preset threshold, one chromaticity correction model is optionally selected as a final chromaticity correction model; when an absolute value of a difference between the two is greater than or equal to a preset threshold value, the two are compared with a standard chromaticity of the selected reference substance, respectively, and a chromaticity correction model corresponding to a training result having a smaller absolute value of a difference with the standard chromaticity of the selected reference substance is selected as a final chromaticity correction model.

A second aspect of the present invention provides a water quality testing device according to claim 8 and configured to perform the above described method, the device including, *inter alia :* a mixing module configured to obtain a test sample, the test sample including a target test substance; an acquisition module, configured to determine a reference substance corresponding to the test sample; a correction model construction module configured to construct a chromaticity correction model based on the reference substance, and further configured to acquire a first chromaticity of the test sample under a first environment; a revision module configured to determine a second chromaticity of the test sample under a second environment based on the first chromaticity and the chromaticity correction model; and a target test substance concentration determining module configured to determine a concentration of the target test substance based on the second chromaticity.

According to the invention, the acquisition module is configured to obtain a third chromaticity of the reference substance under the first environment and a fourth chromaticity of the reference substance under the second environment, and the correction model construction module configured to construct a chromaticity correction model by a neural network algorithm or a multivariate non-linear fitting method according to a chromaticity difference between the fourth chromaticity and the third chromaticity, if the absolute value of the chromaticity difference between the fourth chromaticity and the third chromaticity is greater than a preset deviation threshold.

According to an embodiment, obtaining the test sample containing the target test substance under the first environment includes: judging whether an aqueous solution of the target test substance is colored or not, and judging whether the aqueous solution of the target test substance has an interference color or not by the mixing module; taking the aqueous solution of the target test substance as a test sample if the aqueous solution of the target test substance is colored and has no interference color; selecting a corresponding test reagent according to the target test substance if the aqueous solution of the target test substance is colorless or has an interference color, and fusing the selected test reagent with the aqueous solution of the target test substance to obtain a test sample.

According to an embodiment, determining the third chromaticity of the reference substance under the first environment, and the fourth chromaticity of the reference substance under the second environment includes: acquiring color image information of the reference substance under the second environment, and performing color image information processing according to a preset image algorithm; performing chromaticity extraction on the processed color image information of the reference substance under the second environment to obtain a fourth chromaticity of the reference substance under the second environment; acquiring color image information of the reference substance under a first environment, and performing color image information processing according to a preset image algorithm; performing chromaticity extraction on the processed color image information of the reference substance under the first environment to obtain a third chromaticity of the reference substance under the first environment.

According to an embodiment, the acquisition module is further configured to determine whether the color image information of the reference substance meets a testing requirement, and determine whether the color image information of the test sample meets the testing requirement; in a case where the color image information of the reference substance is determined to meet the testing requirement, and the color image information of the test sample is determined to meet the testing requirement, color image information processing is performed on the color image information of the reference substance and on the color image information of the test sample according to a preset image algorithm.

According to an embodiment, the acquisition module includes: an image acquisition module, configured to acquire color image information of the reference substance under a second environment; and acquire color image information of the reference substance and color image information of the target test substance under a first environment; an image processing module, configured to perform color image information processing on the color image information of the reference substance and the color image information of the target test substance according to a preset image algorithm, and perform chromaticity extraction on the processed color image information.

According to an embodiment, the image acquisition module includes a camera, a cell phone, a camera, a scanner or a monitoring apparatus.

According to an embodiment, the preset image algorithm includes at least: image transformation, key region selection, edge testing, noise reduction, smoothing, and chromaticity enhancement. A third aspect of the present invention provides a computer-readable storage medium according to claim 12.

Through the technical solution provided by the present disclosure, the present disclosure has at least the following technical effects:

the water quality testing method of the present disclosure starts from an actual image result, utilizes the reference substance with the fixed chromaticity to perform correction matching with the laboratory condition, thereby obtaining the required color information quickly and efficiently. The method is simple, the test speed is fast, the additional light source, fixed device and other apparatuses are not needed, the requirements and cost of the photographing method for hardware facilities are reduced, and much of analysis and verification work is completed by the image processing and analysis program. The method has a wide application range, does not depend on specific photographing conditions, has good applicability to different photographing apparatuses, light sources and photographing methods, can be used for various testing and analysis methods related to quantitative photographing, and has low requirements for testing conditions. The testing effect is good, the utilization of chromaticity changes caused by chemical reactions is improved, and the relationship between sensitive chromaticity components and concentration is deeply explored, which is helpful to realize quantitative and accurate determination of the concentration.

Other features and advantages of the present disclosure will be described in detail in the Detailed Description section that follows.

## Brief Description of Drawings

The invention is defined by the appended claims. The accompanying drawings are included to provide a further understanding of embodiments of the disclosure and constitute a part of this specification, and together with the detailed description below serve to explain, but not limit, the embodiments of the disclosure. In the drawings:

FIG. 1 is a flow chart of a water quality testing method provided by an embodiment of the present disclosure;
FIG. 2 is a schematic diagram of a water quality testing device provided by an embodiment of the present disclosure;
FIG. 3 is a graph of a chromaticity-concentration mapping relationship, i.e. a standard curve, of a target test substance according to an embodiment of the present disclosure;
FIG. 4 is a schematic diagram of the chromaticity correction effect according to an embodiment of the present disclosure;
FIG. 5 is a schematic diagram of comparing the concentrations of a target test substance in a test sample measured before and after chromaticity correction with a national standard method according to an embodiment of the present disclosure;
FIG. 6 is a schematic diagram of the chromaticity correction effect according to another embodiment of the present disclosure; and
FIG. 7 is a schematic diagram of the chromaticity correction effect according to yet another embodiment of the present disclosure.

## Detailed Description of the Embodiments

A detailed description of embodiments of the disclosure will now be described with reference to the accompanying drawings. It should be understood that the specific embodiments described herein are intended only to illustrate and explain the embodiments of the present disclosure, and are not intended to limit the embodiments of the present disclosure.

It should be noted that the embodiments of the present disclosure and the features in the embodiments can be combined with each other without conflict.

In the present disclosure, location terms such as "up, down, top, bottom", without explanation to the contrary, are generally used to describe the relative positional relationship of components to one another with respect to the direction shown in the drawings or with respect to the vertical, perpendicular, or gravitational direction.

The research of small, portable, automatic, continuous, simple and fast monitoring techniques has been paid more and more attention. It is an important research and application trend in the field of water quality testing to combine optical analysis methods such as chromaticity, gray scale and turbidity with a portable water quality testing apparatus, which can give full play to the advantages of intelligence, miniaturization, automation, integration and portability, and has broad application prospects. For example, in recent years, with the development of cameras, cell phones or other mobile terminals with the photographing function, photographing and result analysis of portable water quality testing apparatuses such as microfluidic chips and paper microfluidic chips have been widely concerned and studied. On one hand, the convenience of the photographing apparatus is highly matched with the portability of the testing apparatus, and on the other hand, intelligent apparatuses with high-resolution cameras and high-speed computing capabilities provide a strong guarantee for the accurate measurement of substances to be tested. The key of quantitative testing of water target substances by the optical method is to identify the color information quickly and accurately. However, the differences in apparatus hardware, camera settings, field light source conditions, photographing angles/distances, etc. can lead to far different photographing results for the same system, which greatly limits the accuracy and application range of quantitative photography methods. The solution of the disclosure is to develop the testing technology which does not depend on the fixed hardware condition. Starting from the image processing technology, the disclosure develops a simple and convenient water quality testing method based on chromaticity correction and analysis, does not need additional apparatuses or maintains the consistency of photographing conditions, and simultaneously has higher testing accuracy, thus having very important practical significance.

The present disclosure will now be described in detail with reference to the accompanying drawings in conjunction with embodiments.

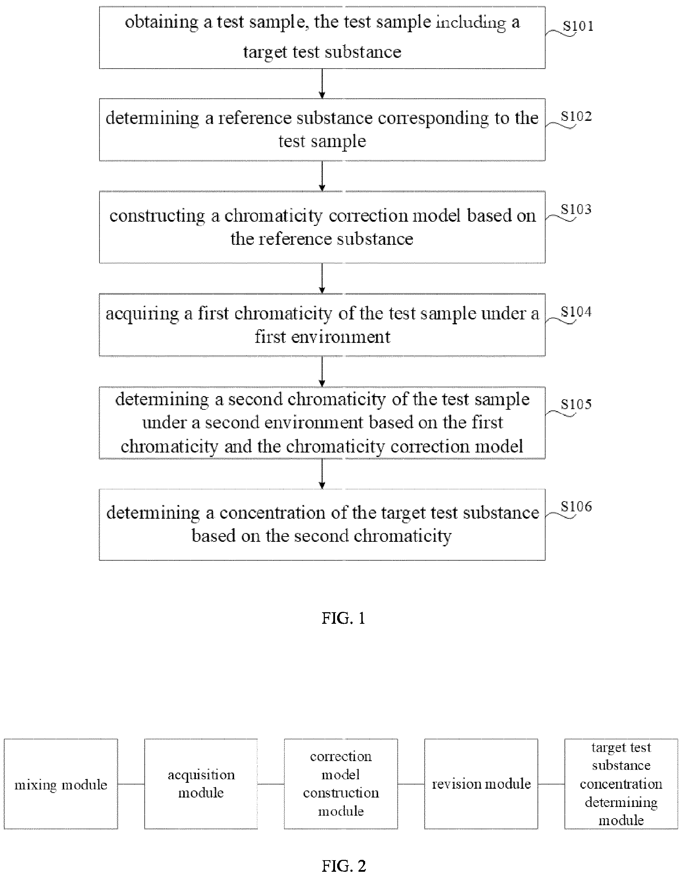

Referring to FIG. 1, an embodiment of the present disclosure provides a water quality testing method, the method including the following steps: S101: a test sample is obtained, the test sample including a target test substance; S102: a reference substance corresponding to the test sample is determined; S103: a chromaticity correction model is constructed based on the reference substance; S104: a first chromaticity of the test sample under a first environment is acquired; S105: a second chromaticity of the test sample under a second environment is determined based on the first chromaticity and the chromaticity correction model; S106: a concentration of the target test substance is determined based on the second chromaticity.

Further, the step that the test sample containing the target test substance is obtained under the first environment includes: whether an aqueous solution of the target test substance is colored or not is judged, and whether the aqueous solution of the target test substance has an interference color or not is judged; the aqueous solution of the target test substance is taken as a test sample if the aqueous solution of the target test substance is colored and has no interference color; a corresponding test reagent is selected according to the target test substance if the aqueous solution of the target test substance is colorless or has an interference color, and the selected test reagent is fused with the aqueous solution of the target test substance to obtain a test sample.

Specifically, in the embodiment of the present disclosure, it can be seen from the above that the water quality testing method provided by the present disclosure performs chromaticity analysis on the water sample to be tested containing the target test substance. Because there is a positive correlation between the concentration of the target test substance and the chromaticity size, the concentration of the target test substance can be converted according to the chromaticity size. Therefore, under this method, it is necessary to ensure that there are colors that can be subjected to chromaticity extraction in the tested water body. Therefore, when there are other soluble substances in the monitored water sample, some soluble substances may have color reaction, and the naked eye can judge whether there are harmful substances through the color shade. However, many substances will not appear abnormal colors when dissolved, and it is impossible to judge the existence or content of harmful substances by naked eyes. Even if there are soluble substances having the color reaction, it is also easily disturbed by similar color reaction soluble substances, and the content cannot be judged by the color shade. In this case, it is necessary to display the target test substance specially, that is, to filter out other interference information and ensure that the final color development is only affected by the target test substance. If the target test substance to be detected in the water sample to be tested dissolves in water and has a single color directly, chromaticity extraction can be performed directly. In this case, the water sample to be tested can be directly used as the test sample. If the water sample to be tested containing the target test substance is colorless or has other interference colors, it is necessary to dye the target test substance, and the dyeing result is only related to the target test substance, that is, the selected test reagent for dyeing only has the color reaction with the target test substance in the test water sample. For example, chromium ions dissolved in water cannot be observed by naked eyes, but after contact with diphenylcarbazide, the chromium ions will undergo the color reaction and show a red color. Therefore, when diphenylcarbazide contact is selected as the test reagent, and then the monitoring water sample is contacted with diphenylcarbazide, a red sample will be obtained. Because the shade of the red color is positively correlated with the content of chromium ions, the content of chromium ions can be quantitatively analyzed by analyzing the shade of the red color of the sample.

Further, the step that the chromaticity of the reference substance under the second environment and the chromaticity of the reference substance under the first environment are acquired includes: color image information of the reference substance is acquired under the second environment, and color image information processing is performed according to a preset image algorithm; chromaticity extraction is performed on the processed color image information of the reference substance under the second environment to obtain a chromaticity of the reference substance under the second environment; color image information of the reference substance is acquired under a first environment, and color image information processing is performed according to a preset image algorithm; chromaticity extraction is performed on the processed color image information of the reference substance under the first environment to obtain a chromaticity of the reference substance under the first environment.

Specifically, the reference substance is photographed in a laboratory to obtain color image information of the reference substance under the second environment, and then the obtained color image information is processed to ensure convenience of subsequent chromaticity extraction. Preferably, the color image processing is performed by image transformation, key region selection, edge testing, noise reduction, smoothing and chromaticity enhancement to obtain clean and single color image information. For example, when performing edge testing, edge testing is performed by using the Canny operator, the edges in the image mainly have the following types: thin line edges, abrupt edges and gradual edges, wherein the abrupt edges can detect first-order differential extreme points, second-order differential 0-crossing points, the thin line edges can detect first-order differential 0-crossing points, second-order differential extreme points, while the gradual edges are harder to test, the second-order differential information is slightly more than the first-order differential. After the edge testing image is obtained by the Canny operator, the improvement of the image quality by the median filtering noise reduction and smoothing algorithm is performed on this basis. Then, chromaticity extraction is performed on the processed color image information summary to obtain the chromaticity of the reference substance under the second environment. Test apparatus material screening, structural design, operation flow design, etc. are performed according to reaction system characteristics. The photographing apparatus includes cameras, cell phones, cameras, scanners, monitors, and other designs that can obtain color information.

The reference substance is then photographed at a photographing site of a to-be-tested substance containing the target substance, and color image information of the reference substance under the first environment is obtained. The color image information of the reference substance under the first environment is first subjected to image processing, and then the color map extraction is performed, as in the chromaticity extraction of the reference substance under the second environment described above. There is no hard requirement on photographing conditions during photographing, but optimization of photographing conditions helps to improve the accuracy of results, such as keeping the level of the reference substance, keeping proper photographing angle and distance of the cell phone, and not too high or too low illuminance of the light source, etc.

Further, the step that the chromaticity correction model is constructed based on the chromaticity of the reference substance under the second environment and the chromaticity of the reference substance under the first environment includes: a chromaticity correction model is constructed according to a chromaticity difference between a chromaticity of the reference substance under the second environment and a chromaticity of the reference substance under the first environment if an absolute value of a chromaticity difference between the chromaticity of the reference substance under the second environment and the chromaticity of the reference substance under the first environment is greater than a preset deviation threshold.

A difference is made between the chromaticity of the reference substance under the second environment and the chromaticity of the reference substance under the first environment, and then the absolute value of the difference is taken. The obtained absolute value of the chromaticity difference is compared with a preset deviation threshold, in a case where the absolute value of the chromaticity difference is greater than the set value, the chromaticity correction model is constructed based on the difference between the chromaticity of the reference substance under the second environment and the chromaticity of the reference substance under the first environment, i.e., the chromaticity correction model is constructed based on a correction path that corrects the chromaticity of the reference substance under the first environment to the chromaticity of the reference substance under the second environment. Preferably, the construction of the chromaticity correction model includes: the chromaticity correction model is constructed by a neural network algorithm or a multivariate non-linear fitting method. Based on the above, a conversion relationship between the chromaticity of the reference substance under the first environment and the chromaticity of the reference substance under the second environment is obtained.

Preferably, the chromaticity correction model is constructed based on a plurality of reference substances, and the selected reference substances are the same type of reference substances. The establishment of the chromaticity correction model is to correct the testing site environment and the laboratory environment, so as to test water quality at anytime and anywhere. Through the chromaticity correction model, the chromaticity of the collected test sample is corrected to the chromaticity under the second environment to ensure the accuracy of testing. The purpose of the reference substance is to obtain the difference of environmental parameters between the testing site and the laboratory environment. If only one reference substance is selected, the accidental error may be very large, and the reference value of a single reference substance is limited, so the final chromaticity correction model may have a large deviation. In order to improve the accuracy of the chromaticity correction model, it is preferable to select a plurality of reference substances simultaneously to construct the chromaticity correction model, these reference substances are of the same type. Because the error judgment of different reference substances will be different, if the correction information of various reference substances is put in the same correction system, the final chromaticity correction model will have a great deviation from the actual situation. The color image information of a plurality of reference substances is collected simultaneously when the image acquisition of the to-be-tested region of the reference substance and the laboratory condition image acquisition are performed. For example, when a standard color card is selected as the reference substance, the same color card includes multiple colors, and these colors are regularly arranged in different regions. When image collection is performed, the color information of all regions of a color card is collected, and then in the post-processing, a plurality of contrast relationships are formed automatically corresponding to the region to be built in each color region and the image information under the second environment. After obtaining all the corresponding relationships, they are sorted into contrast sets, and the chromaticity correction model is constructed based on the contrast sets.

Further, the step that the chromaticity correction model is constructed includes: a chromaticity correction model is constructed by a neural network algorithm or a multivariate non-linear fitting method.

Specifically, as the application of artificial intelligence becomes more widespread, the use of artificial intelligence to construct the image correction model in the solution of the present disclosure can lead to considerable progress in the construction time of the accuracy of the correction model. Preferably, the present disclosure utilizes a neural network algorithm or a multivariate non-linear fitting method to construct the correction model. Among them, it is preferable to use the BP neural network for correction model construction, since BP neural network is currently applied widely and model stability has been verified. The BP neural network is a multi-layer feed-forward network trained by backpropagation of errors, the algorithm of which is called BP algorithm, and its basic idea is a gradient descent method using a gradient search technique in order to minimize the mean square error of the errors between the actual output values and the desired output values of the network. First, a BP network is constructed, the BP network including an input layer, a hidden layer and an output layer, each layer including a plurality of neurons, denoted as n, p, q. Here, the neurons are a topological network established by biological studies and response mechanisms of the brain, which simulates the process of nerve conflict. The ends of multiple dendrites receive external signals and transmit them to neurons for processing and fusion, and finally the nerves are transmitted to other neurons or effectors through axons. First, the color image information of a plurality of reference substances in the region to be tested is collected, and after graphic processing and chromaticity extraction, the chromaticities of all reference substances in the region to be tested are used as training samples and input into the output layer, and recorded as an input vector set $x = (x_1, x_2, ..., x_n)$. Then the k-th input sample and the corresponding desired output are randomly selected, and are denoted as:

$$x(k) = \left( x_1(k), x_2(k), \cdots, x_n(k) \right)$$

$$d_o(k) = \left( d_1(k), d_2(k), \cdots, d_o(k) \right)$$

wherein $d_o$ is a desired output vector, $d_o = (d_1, d_2, ..., d_q)$. Then the input and output of each neuron of the hidden layer are obtained by calculating, according to the above result, the partial derivative solving is performed, a partial derivative $\delta_o(k)$ is obtained, and then the connection weight correction between the hidden layer and the output layer is performed, and the correction formula is:

$$\Delta w_{ho}(k) = -\mu \frac{\partial e}{\partial w_{ho}} = \mu \delta_o(k) ho_h(k)$$

wherein $w_{ho}(k)$ is a connection weight between the hidden layer and the output layer; $h_o$ is an output variable for the hidden layer, $h_o = (d_{o1}, d_{o2}, ..., d_{op})$. The connection weight correction is performed between the input layer and the hidden layer, and the correction formula is:

$$\Delta w_{ih}(k) = -\mu \frac{\partial e}{\partial w_{ih}} = \delta_h(k) x_i(k)$$

wherein $w_{ih}(k)$ is a connection weight between the input layer and the hidden layer. Finally, based on the connection weight, a global error is calculated, and the calculation formula is:

$$E = \frac{1}{2m} \sum_{k=1}^{m} \sum_{o=1}^{q} \left( d_o(k) - y_o(k) \right)^2$$

The BP network formally continually shrinks the global error in order to achieve that the chromaticity of the to-be-tested site is infinitely close to the laboratory chromaticity, because it cannot be guaranteed that the chromaticity of the to-be-tested region completely meets the laboratory condition chromaticity, the iteration times or the global error standard is preset, when the iteration times are completed or the preset global error standard is reached, iteration is stopped, the correction model is output. Therefore, judgement is made after each iteration is completed, and if the iteration result does not meet the requirements, the input and output of the hidden layer are recalculated, and iteration is performed again until the iteration requirements are met, and the correction model is output.

In another possible embodiment, affected by the environment, different environments of the region to be tested may be adapted to different calibration models. The correction model is constructed by the neural network algorithm and the multivariate non-linear fitting method according to adaptive training, all of which can provide the correction model meeting the requirements. But in order to further improve the correction effect, it is preferable to perform training of the BP neural network correction model and training of the fitting correction model once each time, and then compare the two correction models, and select a correction model with a higher accuracy rate as the correction model of the current test sample to improve the correction effect. Specifically, the chromaticity correction model construction is performed using the neural network algorithm and the multivariate non-linear fitting method respectively, and based on a certain selected reference substance, the two constructed chromaticity correction models are trained respectively, and training results of the two chromaticity correction models are obtained; the training results of the two chromaticity correction models are compared: when the absolute value of the difference between the two is less than a preset threshold, one chromaticity correction model is optionally selected as the final chromaticity correction model; when the absolute value of the difference between the two is greater than or equal to the preset threshold value, the two are compared with the standard chromaticity of the selected reference substance, respectively, and the chromaticity correction model corresponding to a training result having a smaller absolute value of the difference with the standard chromaticity of the selected reference substance is selected as a final chromaticity correction model.

Further, the step that the chromaticity of the test sample under the first environment is acquired includes: the color image information of the test sample is acquired under the first environment, and color image information processing is performed according to the preset image algorithm; chromaticity extraction is performed on the processed color image information of the test sample under the first environment to obtain the chromaticity of the test sample under the first environment.

Specifically, after obtaining the chromaticity correction model, chromaticity conversion of the to-be-tested sample can be performed based on the chromaticity correction model, i.e., the chromaticity conversion of the test sample in the to-be-tested region and laboratory conditions can be performed. First, a color image of the sample to be tested is collected at the water quality testing site, and then in accordance with the reference color image processing method, image processing is performed, and then the chromaticity of the test sample under the first environment is acquired in the processed color image. The chromaticity of the test sample under the first environment is taken as a known condition and then is led into the chromaticity correction model obtained above, and the corresponding target substance concentration can be determined using the conversion relationship and chromaticity-concentration mapping relationship, in the following referred to as the standard curve, included in the chromaticity correction model.

Further, before performing color image information processing on the color image information of the reference substance and on the color image information of the test sample according to the preset image algorithm, the method further includes: the testing requirement includes at least: image pixels, illumination intensity and light uniformity of a color image. It is determined that the color image information of the reference substance meets the testing requirement, and it is determined that the color image information of the test sample meets the testing requirement.

Specifically, during the color image acquisition process, it is possible to cause abnormalities in the acquired color image information even under the assured preferred acquisition method, due to apparatus reasons or other emergencies. The abnormal color image information, even after image processing, cannot extract the correct chromaticity. If the image pixels of the water quality testing site are too small, the camera resolution is low or the photographing distance is too far, resulting in a lack of the amount of information needed for testing. Whether the illumination intensity meets the requirements is analyzed, too high or too low illumination intensity at the site for water quality testing causes too high proportion of noise information, and severely interferes with recognition of the information to be tested. It is necessary to adjust the light conditions or settings such as exposure time, sensitivity of the photographing apparatus. Light uniformity also has an effect on data accuracy, light unevenness is very likely to cause large differences in different sets of photographing conditions, it is also possible to have large deviations in chromaticity information at different positions of the water quality testing site during a single photographing process in severe cases, and if this problem occurs, it is necessary to adjust the light source condition, the position of the substance to be tested, the photographing angle, and the like. In order to avoid this, it is preferable that, after obtaining the color image information and before performing the color image information processing, the obtained original color image information is first subjected to testing judgment, i.e., judging whether the obtained image information meets testing standards, which include a preset image pixel size, an illumination intensity value and a light uniformity degree. For example, the Retinex algorithm is used to perform illumination intensity and uniformity analysis for illumination intensity judgement and light uniformity judgement.

Specifically, an Retinex algorithm basic model is a color constancy-based color theory developed with the human visual system as a starting point, which considers that the human eye's perception of an object's color is closely related to the reflective properties of the object's surface, i.e., objects with low reflectivity appear darker and objects with high reflectivity appear lighter; the human eye's perception of object color is consistent and is not affected by illumination changes. In this study, an illumination component is estimated from the original image by establishing the illumination reflection model of the image, so as to judge whether the illumination intensity of the photographed image meets the requirements. The calculation formula for the Retinex algorithm to estimate the illumination component is:

$$i_c^{'}\left(x,y\right) = F\left(x,y\right) * f_c\left(x,y\right), C \in \left\{R,G,B\right\}$$

wherein $i_c^{'}(x,y)$ is the illumination component; $F(x,y)$ is a center wrap-around function, which is defined as:

$$F\left(x,y\right) = K \cdot e^{-\frac{x^2+y^2}{\sigma^2}}$$

$$\iint F\left(x,y\right) dxdy = 1$$

wherein $\sigma$ is a standard deviation, denotes a scale constant of the Gaussian wrap-around function, and determines the range of action of a convolution kernel. The center wrap-around Retinex method is mainly divided into a single-scale method and a multi-scale method, the single-scale method computes an illumination component estimate value $i_c^{'}(x,y)$ of the C-th color channel, and then by logarithmic transformation:

$$\ln\left[f_c\left(x,y\right)\right] = \ln\left[i_c\left(x,y\right) \cdot r_c\left(x,y\right)\right] = \ln\left[i_c\left(x,y\right)\right] + \ln\left[r_c\left(x,y\right)\right]$$

a reflection component is then calculated, thus obtaining Retinex data:

$$R_c\left(x,y\right) = \ln\left[r_c^{'}\left(x,y\right)\right] = \ln\left[f_c\left(x,y\right)\right] - \ln\left[i_c^{'}\left(x,y\right)\right]$$
$$= \ln\left[f_c\left(x,y\right)\right] - \ln\left[F\left(x,y\right) * f_c\left(x,y\right)\right]$$

By the above method, after judging that the color image information meets the testing requirement, the color image information processing is performed according to a preset image algorithm, and the validity of the subsequent processing information is guaranteed.

Further, the method further includes: the pre-set standard curve, i.e. a chromaticity-concentration mapping relationship, is constructed, including: a plurality of aqueous solutions containing the target test substance are selected, wherein the concentration of the target test substance in each aqueous solution is known and different; chromaticities of the plurality of aqueous solutions including the target test substance under the second environment are acquired; a function relationship between the concentration of the target test substance and the chromaticity of the aqueous solution of the target test substance under the second environment is obtained based on the concentration of the target test substance in each aqueous solution and the corresponding chromaticity of each aqueous solution under the second environment, and the pre-set standard curve is obtained from the function relationship.

Specifically, it is known that the chromatic value of the test sample is positively correlated with the concentration of the corresponding target test substance, and in the construction of the standard curve, a plurality of aqueous solutions of the target test substance having known concentrations are selected, the aqueous solutions of the target test substance having different concentrations and different chromatic values. Then, chromaticity extraction is performed on each aqueous solution of the target test substance in the laboratory, and the extracted chromaticity is brought into a one-to-one relationship with the corresponding concentration of the target test substance. Then a plurality of correspondences can be obtained, a functional relationship of the concentration of the target test substance and the chromaticity is collated according to the plurality of correspondences, and then the standard curve is drawn according to the functional relationship. The standard curve is the correspondence between the chromaticity of the test sample of the target test substance under the second environment and the concentration of the target test substance.

Further, the step that the concentration of the target test substance in the test sample is obtained according to the preset standard curve includes: the chromaticity of the test sample under the first environment is corrected to the chromaticity of the test sample under the second environment using the chromaticity correction model; the concentration of the target test substance corresponding to the chromaticity of the test sample containing the target test substance under the second environment is found according to the preset standard curve, and the concentration of the target test substance in the test sample is determined.

Specifically, after obtaining the chromaticity of the test sample under the first environment, according to the obtained chromaticity correction model, the chromaticity of the test sample under the first environment is corrected to the chromaticity under the second environment, and then the concentration of the target test substance corresponding to the chromaticity under the test sample containing the target test substance under the second environment is found based on the standard curve constructed as described above, which is the concentration of the target test substance in the test sample.

In a case where an absolute value of a chromaticity difference between the chromaticity of the reference substance under the first environment and the chromaticity of the reference substance under the second environment is less than or equal to a preset deviation threshold, the chromaticity of the test sample under the first environment is acquired; the concentration of the target test substance in the test sample is obtained according to a preset standard curve and the chromaticity of the test sample under the first environment.

The water quality testing method of the present disclosure starts from an actual image result, utilizes the reference substance with the fixed chromaticity to perform correction matching with the laboratory condition, thereby obtaining the required color information quickly and efficiently. The method is simple, the test speed is fast, the additional light source, fixed device and other apparatuses are not needed, the requirements and cost of the photographing method for hardware facilities are reduced, and much of analysis and verification work is completed by the image processing and analysis program. The method has a wide application range, does not depend on specific photographing conditions, has good applicability to different photographing apparatuses, light sources and photographing methods, can be used for various testing and analysis methods related to quantitative photographing, and has low requirements for testing conditions. The testing effect is good, the utilization of chromaticity changes caused by chemical reactions is improved, and the relationship between sensitive chromaticity components and concentration is deeply explored, which is helpful to realize quantitative and accurate determination of the concentration.

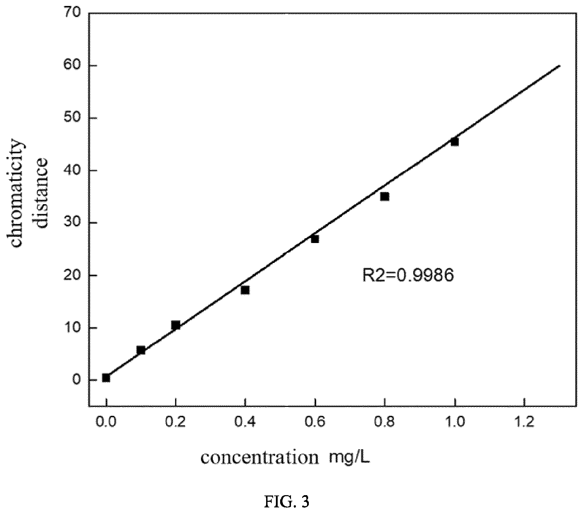

Embodiment 1: please refer to FIG. 3, taking the detection of chromium ions in water as an example, after the determination of the compound reagent reaction system mainly composed of diphenylcarbazide, and paper microfluidic chip structure design, a standard curve, i.e. a chromaticity-concentration mapping relationship, is constructed and correlation fitting between a chromium ion concentration and a chromaticity distance is performed under the second environment to obtain the base data for the standard curve. For this system, the chromaticity distance is proportional to the chromium ion concentration in a testing linear interval. A microfluidic chip added with different colored dyes is selected as the reference substance, and the reference substance photographing is performed in the laboratory optical environment where the standard curve is obtained so as to obtain the laboratory standard chromaticity of the reference substance image.

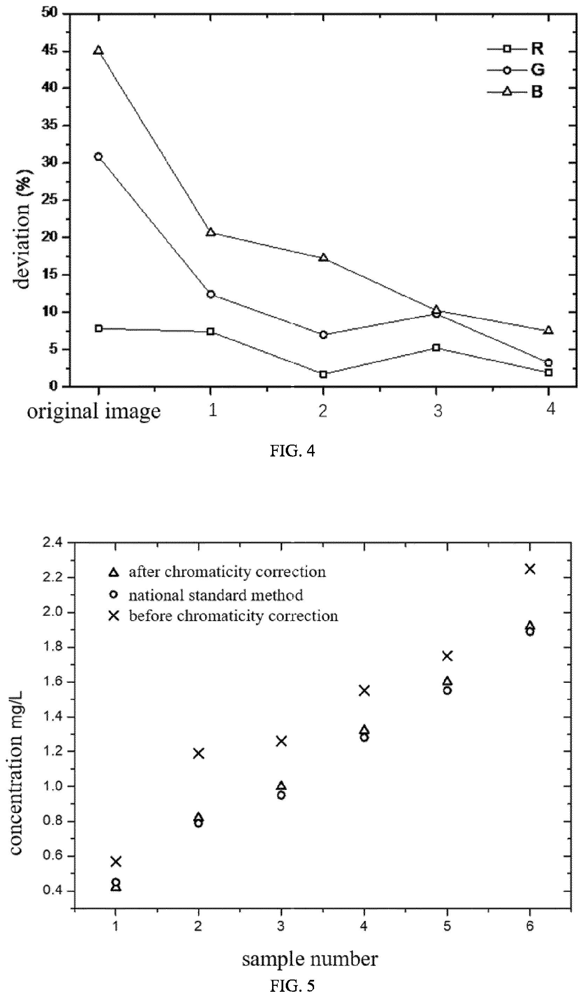

The reference substance is photographed and image processed under light conditions on the water quality testing site. The image result is analyzed to judge whether the photographing meets the analysis requirements. The actual chromaticity of the reference substance is compared with the laboratory standard chromaticity of the reference substance under experimental conditions, it is found that the required chromaticity component deviates greatly from the laboratory result, corrected matching of the on-site image chromaticity with the laboratory chromaticity is performed on both the color card and the chip, and it is determined that the chromaticity correction model is determined by using an optimal multivariate non-linear fitting method. In order to compare the obtained correction effects, various kinds of chromaticity correction model training are respectively performed, first, the standard color card is used as the reference substance, and the chromaticity correction is performed once by conventional chromaticity correction, and then the dyed paper microfluidic chip is used as the reference substance, and the correction is performed by methods of BP neural network and fitting, respectively, and the correction effects are obtained respectively. Finally, the aqueous solution containing the target test substance is configured, and then the solution is used as a reference substance, and the chromaticity correction is performed once, and the corresponding correction effect is obtained, please refer to FIG. 4 for comparison of the correction effects, 1 represents that the reference substance is a standard color card, and the correction method is the correction effect of the BP neural network algorithm; 2 represents that the reference substance is a dyed paper microfluidic chip, and the correction method is the correction effect of the multivariate non-linear fitting method; 3 represents that the reference substance is a dyed paper microfluidic chip, and the correction method is the correction effect of the BP neural network algorithm; 4 represents that the reference substance is a solution of the same type as the test sample, and the correction method is the calibration effect of the BP neural network algorithm. The results show that the average deviation between the actual chromaticity of the reference substance and the laboratory standard chromaticity of the reference substance can be greatly reduced to be less than 5% by the conversion relationship established by this method.

An on-site testing experiment is further conducted, reacting and optically testing is performed on the chromium containing system. Operations such as key region selection, edge testing, noise reduction smoothing, chromaticity enhancement are performed on the image, and finally respective chromaticity component values are obtained, the actual chromium ion concentration can be obtained by matching the on-site chromaticity component photographed on-site and the standard curve. Please refer to FIG. 5., the chromium ion concentration obtained by this method is compared with the national standard method, the average deviation is less than 7%, and if the image chromaticity correction is not performed, the average deviation is more than 15%.

Embodiment 2: taking the detection of nickel ions in water as an example, reaction system determination is first performed: for the property of the target to be tested, a paper microfluidic chip is chosen for testing, a core portion of the chip is a filter paper which is hydrophobically modified (a sample inlet, a channel, and a test pool are hydrophilic, and a remaining region is hydrophobic, the test pool is preloaded with a complex reagent which containing dimethylglyoxime as a main substance and is capable of producing specific color reaction with nickel), in testing, a nickel-containing aqueous sample is added to the paper microfluidic chip from a sample application zone, and the sample flows along the channel to the test pool and reacts with the reagent to form a pink substance.

Correlation fitting of the nickel ion concentration to the chromaticity distance is performed under the second environment to obtain base data for a standard curve, for which the chromaticity distance is proportional to the nickel ion concentration in the testing linear interval. A standard color card and a microfluidic chip which are added with different colored dyes are selected as the reference substances, and reference substance photographing is performed in a laboratory optical environment where a standard curve is obtained so as to obtain the laboratory standard chromaticity of the reference substance image.

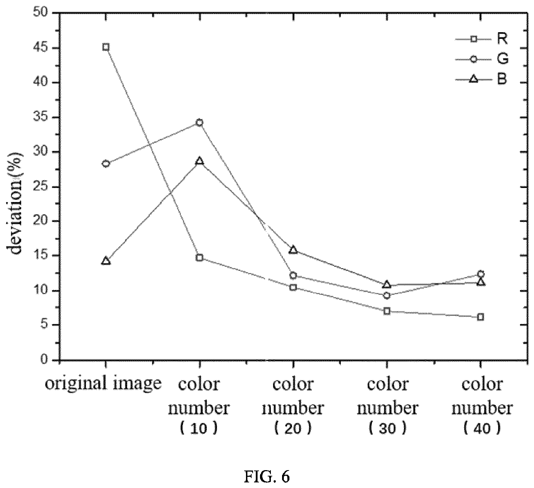

The reference substance is photographed under the light conditions on the water quality monitoring site, and the on-site chromaticity component is obtained. By comparing the on-site chromaticity component with the laboratory standard chromaticity of the reference substance, it is found that there is a big deviation between the actual chromaticity of the reference substance and the laboratory standard chromaticity of the reference substance, so chromaticity correction is needed. As known above, when the correction model is constructed by the BP neural network, the chrominance obtained from the to-be-tested region of the reference substance is taken as the training sample set, and the accuracy of the correction model is related to the training sample set and the iteration times. Under the condition that the preset iteration times are kept unchanged and the construction time of the correction model is short enough, the accuracy of the correction model can be effectively improved by increasing the size of the training sample set. In order to obtain the optimal training sample set size, that is, to obtain the optimal color number of the reference substance, preferably, reference substances with different color numbers are selected respectively, and correction model is constructed respectively, and then the chromaticity correction effect of the reference substance is obtained. The color numbers are 10, 20, 30 and 40 respectively. Please refer to FIG. 6., the results show that the richer the color information of the reference substance, the better the correction effect of the correction model, and with the increasing number of colors, the smaller the improvement of the correction effect. In order to improve the efficiency of correction model construction and reduce the workload of reference substance selection, 30 or 40 kinds of color information are preferred.

An on-site testing experiment is further conducted, reacting and optically testing is performed on the nickel containing system to obtain each chromaticity component value, and the result of the on-site chromaticity component photographed on-site is matched with the standard curve, i.e., the actual concentration of nickel ions is obtained. The nickel ion concentration obtained by the present method is compared with the national method, the deviation is less than 9%, and if the image chromaticity correction is not performed, the average deviation is greater than 25%, thus verifying the reliability of the present method, and at the same time, the present disclosure has significant advantages in terms of testing time, convenience and the like.

Embodiment 3: taking the detection of chromium ions in water as an example, a microfluidic chip is used to perform testing, and the structures of a channel, a testing pool, etc. are constructed on the chip substrate and then encapsulated with a cover sheet to finally form a closed space. A compound reagent mainly composed of dibenzoyldihydrazide is embedded in part of the channel and the testing pool of the substrate, and is capable of producing specific color reaction with chromium. Correlation fitting between a chromium ion concentration and a chromaticity distance is performed under the second environment to obtain the base data for the standard curve. For this system, the chromaticity distance is proportional to the chromium ion concentration in a testing linear interval. A microfluidic chip added with different colored dyes is selected as the reference substance, and the reference substance photographing is performed in the laboratory optical environment where the standard curve is obtained so as to obtain the laboratory standard chromaticity of the reference substance.

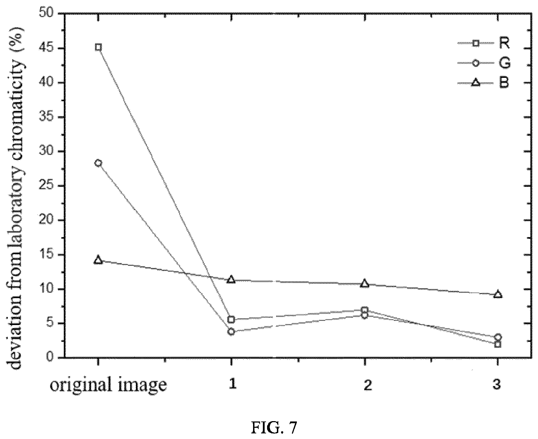

The reference substance is photographed under light conditions on a certain device site to obtain the actual chromaticity of the reference substance. The actual chromaticity of the reference substance is compared with the laboratory standard chromaticity of the reference substance, it is found that the required chromaticity component deviates greatly from the laboratory result, and the correction effects of chromaticity correction by the multivariate non-linear fitting method and the BP neural network algorithm are compared to determine the optimal correction method. Preferably, on the premise of guaranteeing that the reference substance and the chromaticity information are consistent, correction model construction is performed using the multivariate non-linear fitting method and the BP neural network algorithm, respectively, chromaticity correction is performed according to the constructed correction model, and then the correction effects are compared. The reference substance type is then changed to change the dyed paper microfluidic chip to the same type of aqueous solution as the test sample, and then chromaticity correction is performed, and the correction effect is taken as the desired effect. The correction effect comparison between the multivariate non-linear fitting method and the BP neural network algorithm and also the correction effect comparison between each of the multivariate non-linear fitting method and the BP neural network algorithm and the ideal correction effect are obtained, respectively, please refer to FIG. 7, 1 represents that the reference substance is the dyed paper microfluidic chip, and the correction method is the correction effect of the multivariate non-linear fitting method; 2 represents that the reference substance is a dyed paper microfluidic chip, and the correction method is a correction effect of the BP neural network algorithm; 3 represents that the reference substance is a solution of the same type as the test sample, and the correction method is the calibration effect of the BP neural network algorithm. The results show that in the present embodiment, the correction effect is better using the multivariate non-linear fitting method, and the deviation from the ideal correction effect is not large, and the method can be used as a correction model for on-site testing.

An on-site testing experiment is further conducted, reacting and optically testing is performed on the chromium containing system. Operations such as key region selection, edge testing, noise reduction smoothing, chromaticity enhancement are performed on the image, and finally respective chromaticity component values are obtained, the actual chromium ion concentration can be obtained by matching the on-site chromaticity component photographed on-site and the standard curve. The chromium ion concentration obtained by this method is compared with the national standard method, the deviation is less than 11%, which verifies the reliability of the method. At the same time, the disclosure has obvious advantages in the aspects of testing time, convenience and the like.

Embodiment 4: taking the detection of hydrogen in air as an example, a metal oxide material is used as a testing reagent to prepare a metal powder by a precipitation method and a hydrothermal method , the powder is milky white and gradually turns dark blue with contact with hydrogen. Under the second environment, correlation fitting between the hydrogen concentration and the chromaticity distance is performed to obtain a standard curve. For this system, the chromaticity distance is proportional to the hydrogen concentration in the testing linear interval. Particles with different typical colors are selected as the reference substance, and the reference substance is photographed in the laboratory optical environment where the standard curve is obtained, and the laboratory standard chromaticity of the reference substance is obtained.

The reference substance is photographed under the light conditions on the testing site to obtain the actual chromaticity of the reference substance. The actual chromaticity of the reference substance is compared with the laboratory standard chromaticity of the reference substance, it is found that the actual chromaticity of the reference substance deviates greatly from the laboratory standard chromaticity of the reference substance. The neural network algorithm is used to correct and match the actual chromaticity of the reference substance and the laboratory standard chromaticity of the reference substance. The results show that the average deviation between the on-site photographing chromaticity and the laboratory standard chromaticity can be greatly reduced to be less than 5% by the conversion relationship established by this method.

An on-site testing experiment is further conducted, the configured hydrogen with the concentrations of 1%, 2%, 4% and 10% is tested to obtain each chromaticity component value, the on-site chromaticity component shot on the site is matched with the standard curve to obtain the calculated hydrogen concentration, and the deviations from the real value are 8%, 6%, 3%, 4% and 1% respectively. If the image chromaticity correction is not performed, the average deviation of the on-site chromaticity component is greater than 20%, the reliability of the method is verified. Meanwhile, the disclosure has obvious advantages in testing time (less than 1 minute), convenience and the like.

Referring to FIG. 2, a second aspect of the present disclosure provides a water quality testing device, including: a mixing module configured to obtain a test sample containing a target test substance under a first environment; an acquisition module configured to select a reference substance according to the test sample, and acquiring the chromaticity of the reference substance under a second environment and the chromaticity of the reference substance under the first environment, respectively; and obtaining a chromaticity of the test sample under the first environment; a correction model construction module, configured to construct a correction model based on the chromaticity of the reference substance under the second environment and the chromaticity of the reference substance under the first environment; a revision module configured to obtain a chromaticity of the test sample under the second environment according to the chromaticity of the test sample under the first environment and the correction model; a target test substance concentration determining module configured to obtain a concentration of the target test substance in the test sample according to a preset standard curve and the chromaticity of the test sample under the second environment; wherein the preset standard curve is a corresponding relationship between the chromaticity of the test sample containing the target test substance under the second environment and the concentration of the target test substance.

Further, the step that the test sample containing the target test substance is obtained under the first environment includes: the mixing module judges whether an aqueous solution of the target test substance is colored or not, and judges whether the aqueous solution of the target test substance has an interference color or not; the aqueous solution of the target test substance is taken as a test sample if the aqueous solution of the target test substance is colored and has no interference color; a corresponding test reagent is selected according to the target test substance if the aqueous solution of the target test substance is colorless or has an interference color, and the selected test reagent is fused with the aqueous solution of the target test substance to obtain a test sample.

Further, the step that the chromaticity of the reference substance under the second environment and the chromaticity of the reference substance under the first environment are respectively acquired includes: the acquisition module acquires color image information of the reference substance under the second environment, and performs color image information processing according to a preset image algorithm; chromaticity extraction is performed on the processed color image information of the reference substance under the second environment to obtain a chromaticity of the reference substance under the second environment; the acquisition module acquires color image information of the reference substance under the first environment, and performs color image information processing according to a preset image algorithm; chromaticity extraction is performed on the processed color image information of the reference substance under the first environment to obtain a chromaticity of the reference substance under the first environment; the step that the chromaticity of the test sample under the first environment is obtained includes: the acquisition module acquires the color image information of the test sample under the first environment, and performs color image information processing according to a preset image algorithm; chromaticity extraction is performed on the processed color image information of the test sample under the first environment to obtain a chromaticity of the test sample under the first environment.

Further, the step that according to the chromaticity of the reference substance under the second environment and the chromaticity of the reference substance under the first environment, the correction model is constructed includes: a correction model is constructed by the correction model construction module according to a chromaticity difference between a chromaticity of the reference substance under the second environment and a chromaticity of the reference substance under the first environment if an absolute value of a chromaticity difference between the chromaticity of the reference substance under the second environment and the chromaticity of the reference substance under the first environment is greater than a preset deviation threshold.

Further, the acquisition module is further configured to determine whether the color image information of the reference substance meets the testing requirement, and determine whether the color image information of the test sample meets the testing requirement; in a case where the color image information of the reference substance is determined to meet the testing requirement, and the color image information of the test sample is determined to meet the testing requirement, color image information processing is performed on the color image information of the reference substance and on the color image information of the test sample according to a preset image algorithm.

Further, the acquisition module includes: an image acquisition module, configured to acquire color image information of the reference substance under a second environment; and acquire color image information of the reference substance and color image information of the target test substance under a first environment; an image processing module, configured to perform color image information processing on the color image information of the reference substance and the color image information of the target test substance according to a preset image algorithm, and perform chromaticity extraction on the processed color image information.

Further, the image acquisition module includes a camera, a cell phone, a camera, a scanner or a monitoring apparatus.

Further, the preset image algorithm includes at least: image transformation, key region selection, edge testing, noise reduction, smoothing, and chromaticity enhancement.

A third aspect of the disclosure provides a water quality testing system including the water quality testing device as described above.

A fourth aspect of the present disclosure provides a computer-readable storage medium having stored thereon instructions which, when run on a computer, cause the computer to perform the water quality testing method described above.

The preferred embodiments of the present disclosure are described in detail in combination with the drawings, but the present disclosure is not limited to the specific details in the above embodiments. Various simple variations can be made to the technical solutions of the present disclosure in the scope of the technical concept of the present disclosure, and these simple variations all fall within the protection scope of the present disclosure.

In addition, it should be noted that all the specific technical features described in the specific embodiments can be combined in any appropriate mode within the scope of the appended claims, and all possible combination modes will not be described separately in order to avoid unnecessary repetition. In addition, various different embodiments of the present disclosure can also be combined optionally, within the scope of the appended claims.

**Claims**

**1.** A water quality testing method, comprising: obtaining a test sample, the test sample comprising a target test substance; determining a reference substance corresponding to the test sample; determining a third chromaticity of the reference substance under a first environment, and a fourth chromaticity of the reference substance under a second environment;

obtaining a chromaticity-concentration mapping relationship characterizing a mapping relationship between the chromaticity of the test sample under the second environment and the concentration of the target test substance; determining an absolute value of the chromaticity difference between the fourth chromaticity and the third chromaticity, and

o if an absolute value of the chromaticity difference between the fourth chromaticity and the third chromaticity is greater than a pre-set deviation threshold: constructing a chromaticity correction model by a neural network algorithm or a multivariate non-linear fitting method according to a chromaticity difference between the fourth chromaticity and the third chomaticity;

acquiring a first chromaticity of the test sample under the first environment; determining a second chromaticity of the test sample under the second environment based on the first chromaticity and the chromaticity correction model; and determining a concentration of the target test substance based on the determined second chromaticity and the chromaticity-concentration mapping relationship; and

o in a case where the absolute value of the chromaticity difference between the fourth chromaticity and the third chromaticity is less than or equal to the pre-set deviation threshold,

the method further comprises: acquiring the chromaticity of the test sample under the first and obtaining the concentration of the target test substance in the test sample based on the chromaticity-concentration mapping relationship and the chromaticity of the test sample under the first environment, wherein the first environment is a test site environment in which the test sample is located; and the second environment is a laboratory standard environment.

2. The water quality testing method according to claim 1, **characterized in that** obtaining the test sample comprises: judging whether an aqueous solution of the target test substance is colored or not, and judging whether the aqueous solution of the target test substance has an interference color or not; taking the aqueous solution of the target test substance as a test sample if the aqueous solution of the target test substance is colored and has no interference color; selecting a corresponding test reagent according to the target test substance if the aqueous solution of the target test substance is colorless or has an interference color, and fusing the selected test reagent with the aqueous solution of the target test substance to obtain a test sample,
preferably, wherein the test reagent corresponding to the target test substance is a test reagent that undergoes a chromogenic reaction upon fusion with an aqueous solution of the target test substance.

3. The water quality testing method according to claim 1, **characterized in that** determining the third chromaticity of the reference substance under the first environment, and the fourth chromaticity of the reference substance under the second environment comprises: acquiring color image information of the reference substance under the second environment, and performing color image information processing according to a preset image algorithm; performing chromaticity extraction on the processed color image information of the reference substance under the second environment to obtain a fourth chromaticity of the reference substance under the second environment; acquiring color image information of the reference substance under a first environment, and performing color image information processing according to a preset image algorithm; performing chromaticity extraction on the processed color image information of the reference substance under the first environment to obtain a third chromaticity of the reference substance under the first environment,

preferably, wherein the preset image algorithm comprises at least: image transformation, key region selection, edge testing, noise reduction, smoothing and chromaticity enhancement,
more preferably, wherein the color image information chromaticity extraction under the second environment and the color image information chromaticity extraction under the first environment are performed under a same chromaticity system, and the chromaticity system is any one of: RGB, HSV, CMYK, CIE, and LAB.

4. The water quality testing method according to claim 1, **characterized in that** when the chromaticity correction model construction is performed, the chromaticity correction model construction is performed using a neural network algorithm and a multivariate non-linear fitting method respectively, and the two constructed chromaticity correction models are trained respectively based on a certain selected reference substance, and training results of the two chromaticity correction models are obtained; the training results of the two chromaticity correction models are compared: when an absolute value of a difference between the two is less than a preset threshold, one chromaticity correction model is optionally selected as a final chromaticity correction model; when an absolute value of a difference between the two is greater than or equal to a preset threshold value, the two are compared with a standard chromaticity of the selected reference substance, respectively, and a chromaticity correction model corresponding to a training result having a smaller absolute value of a difference with the standard chromaticity of the selected reference substance is selected as a final chromaticity correction model.

5. The water quality testing method according to claim 3, **characterized in that** before performing color image information processing on the color image information of the reference substance according to the preset image algorithm, the method further comprises: determining that the color image information of the reference substance meets a testing requirement, and determining that the color image information of the test sample meets the testing requirement,

preferably, wherein the testing requirement comprises at least: image pixels, illumination intensity and light uniformity of a color image.

6. The water quality testing method according to claim 1, **characterized in that** obtaining a chromaticity-concentration mapping relationship comprises: constructing the chromaticity-concentration mapping relationship comprising: selecting a plurality of aqueous solutions containing the target test substance, wherein the concentration of the target test substance in each aqueous solution is known and different; acquiring chromaticities of the plurality of aqueous solutions comprising the target test substance under the second environment; obtaining a function relationship between the concentration of the target test substance and the chromaticity of the aqueous solution of the target test substance under the second environment based on the concentration of the target test substance in each aqueous solution and the corresponding chromaticity of each aqueous solution under the second environment, and obtaining the chromaticity-concentration mapping relationship from the function relationship.

7. The method according to claim 1, **characterized in that** the reference substance is a standard color card; a plurality of color regions exist on the standard color card, when performing reference substance color degree extraction, chromaticities of all the color regions on the standard color card are simultaneously extracted to obtain a first chromaticity set; the first chromaticity set is subjected to chromaticity correction model construction as a training sample, or
the reference substance is a paper microfluidic chip, a color card, a microchannel chip or a color developing apparatus; the reference substance is colored by any one of the following modes: an intrinsic color of the reference substance, dye addition, addition of a color reaction system.

8. A water quality testing device for use with a reference substance, comprising: a mixing module configured to obtain a test sample, the test sample comprising a target test substance; an acquisition module configured to determine a reference substance corresponding to the test sample, and acquire a first chromaticity of the test sample under the first environment, a third chromaticity of the reference substance under a first environment, and a fourth chromaticity of the reference substance under a second environment; a correction model construction module configured to:

   determine an absolute value of the chromaticity difference between the fourth chromaticity and the third chromaticity,
   compare the determined absolute value of the chromaticity difference to a pre-set deviation threshold, and
   if the absolute value of the chromaticity difference is greater than a preset deviation threshold, construct a chromaticity correction model by a neural network algorithm or a multivariate non-linear fitting method according to a chromaticity difference between the fourth chromaticity and the third chromaticity,
   a revision module configured to determine the second chromaticity of the test sample under a second environment based on the first chromaticity and the chromaticity correction model constructed by the correction model construction module; and
   a target test substance concentration determining module configured to determine a concentration of the target test substance based on a pre-set chromaticity-concentration mapping relationship and on the second chromaticity determined by the revision module,
   wherein in a case where the absolute value of the chromaticity difference between the fourth chromaticity and the third chromaticity is determined to be less than or equal to the pre-set deviation threshold,
   the target test substance concentration determining module further configured to obtain the concentration of the target test substance in the test sample based on the pre-set chromaticity-concentration mapping relationship and the chromaticity of the test sample under the first environment,
wherein the pre-set chromaticity-concentration mapping relationship reflects a mapping relationship between the chromaticity of the test sample under the second environment and the concentration of the target test substance, and
wherein the first environment is a test site environment in which the test sample is located; and the second environment is a laboratory standard environment.

9. The water quality testing device according to claim 8, wherein the mixing module is configured to judge whether an aqueous solution of the target test substance is colored or not, and whether the aqueous solution of the target test substance has an interference color or not, such that, in use, the aqueous solution of the target test substance is taken as a test sample if the aqueous solution of the target test substance is colored and has no interference color; or a corresponding test reagent according to the target test substance is selected if the aqueous solution of the target test substance is colorless or has an interference color, and the selected test reagent is fused with the aqueous solution of the target test substance to obtain a test sample.

10. The water quality testing device according to claim 8, wherein to acquire the third chromaticity of the reference substance under the first environment and the fourth chromaticity of the reference substance under the second environment, the acquisition module is configured to acquire color image information of the reference substance under the second environment, and to perform color image information processing according to a preset image algorithm; to perform chromaticity extraction on the processed color image information of the reference substance under the second environment to obtain the fourth chromaticity of the reference substance under the second environment; to acquire color image information of the reference substance under the first environment, and to perform color image information processing according to a preset image algorithm; to perform chromaticity extraction on the processed color image information of the reference substance under the first environment to obtain the third chromaticity of the reference substance under the first environment,

preferably, the preset image algorithm comprises at least:
image transformation, key region selection, edge testing, noise reduction, smoothing, and chromaticity enhancement.

11. The water quality testing device according to claim 10, **characterized in that**, the acquisition module is further configured to determine whether the color image information of the reference substance meets a testing requirement, and determine whether the color image information of the test sample meets the testing requirement; in a case where the color image information of the reference substance is determined to meet the testing requirement, and the color image information of the test sample is determined to meet the testing requirement, color image information processing is performed on the color image information of the reference substance and on the color image information of the test sample according to a preset image algorithm,

preferably, the acquisition module comprises: an image acquisition module, configured to acquire color image information of the reference substance under a second environment; and acquire color image information of the reference substance and color image information of the target test substance under a first environment; an image processing module, configured to perform color image information processing on the color image information of the reference substance and the color image information of the target test substance according to a preset image algorithm, and perform chromaticity extraction on the processed color image information,
more preferably, the image acquisition module comprises a camera, a cell phone, a camera, a scanner or a monitoring apparatus.

12. A computer-readable storage medium, **characterized in that** the computer-readable storage medium has stored thereon instructions that, when run on the device of any one of claims 8-11, cause the device to perform the water quality testing method according to any one of claims 1-7.

**Patentansprüche**

1. Wasserqualitätstestverfahren, umfassend:

Beschaffen einer Testprobe, wobei die Testprobe eine Zieltestsubstanz umfasst;
Bestimmen einer Referenzsubstanz, die zu der Testprobe korrespondiert; Bestimmen einer dritten Chromazität der Referenzsubstanz unter einer ersten Umgebung und einer vierten Chromazität der Referenzsubstanz unter einer zweiten Umgebung;

Erhalten einer Chromazitätskonzentrations-Zuordnungsbeziehung, die eine Zuordnungsbeziehung zwischen der Chromazität der Testprobe unter der zweiten Umgebung und der Konzentration der Zieltestsubstanz charakterisiert;

Bestimmen eines Absolutwerts der Chromazitätsdifferenz zwischen der vierten Chromazität und der dritten Chromazität, und

• wenn ein Absolutwert der Chromazitätsdifferenz zwischen der vierten Chromazität und der dritten Chromazität größer ist als ein voreingestellter Schwellenwert: Erstellen eines Chromazitätskorrekturmodells durch einen Neuronalnetzwerk-Algorithmus oder ein Multivariates nichtlineares Fitting-Verfahren entsprechend einer Chromazitätsdifferenz zwischen der vierten Chromazität und der dritten Chromazität;

Erfassen einer ersten Chromazität der Testprobe unter der ersten Umgebung; Bestimmen einer zweiten Chromazität der Testprobe unter der zweiten Umgebung auf der Grundlage der ersten Chromazität und des Chromazitätskorrekturmodells; und Bestimmen einer Konzentration der Zieltestsubstanz auf der Grundlage der bestimmten zweiten Chromazität und der Chromazitäts-Konzentrations-Zuordnungsbeziehung; und

• in einem Fall, in dem der Absolutwert der Chromazitätsdifferenz zwischen der vierten Chromazität und der dritten Chromazität kleiner oder gleich dem voreingestellten Schwellenwert für Abweichungen ist,

wobei das Verfahren ferner folgende Schritte aufweist: Erfassen der Chromazität der Testprobe unter der ersten Umgebung; und

Ermitteln der Konzentration der Zieltestsubstanz in der Testprobe auf der Grundlage der Chromazitätskonzentration und der Chromazität der Testprobe unter der ersten Umgebung,

wobei die erste Umgebung eine Testortumgebung ist, in der sich die Testprobe befindet; und die zweite Umgebung eine Laborstandardumgebung ist.

2. Das Wasserqualitätstestverfahren gemäß Anspruch 1, **dadurch gekennzeichnet, dass** das Ermitteln der Testprobe umfasst: Beurteilen, ob eine wässrige Lösung der Zieltestsubstanz gefärbt ist oder nicht, und Beurteilen, ob die wässrige Lösung der Zieltestsubstanz eine Interferenzfarbe aufweist oder nicht; Verwenden der wässrigen Lösung der Zieltestsubstanz als Testprobe, wenn die wässrige Lösung der Zieltestsubstanz gefärbt ist und keine Interferenzfarbe aufweist; Auswählen eines entsprechenden Testreagenzes entsprechend der Zieltestsubstanz, wenn die wässrige Lösung der Zieltestsubstanz farblos ist oder eine Interferenzfarbe aufweist, und Vermischen des ausgewählten Testreagenzes mit der wässrigen Lösung der Zieltestsubstanz, um eine Testprobe zu erhalten, vorzugsweise wobei das zu der Zieltestsubstanz korrespondierende Testreagenz ein Testreagenz ist, das bei der Vermischung mit einer wässrigen Lösung der Zieltestsubstanz eine chromogene Reaktion eingeht.

3. Das Wasserqualitätstestverfahren gemäß Anspruch 1, **dadurch gekennzeichnet, dass** das Bestimmen der dritten Chromazität der Referenzsubstanz unter der ersten Umgebung und der vierte Chromazität der Referenzsubstanz unter der zweiten Umgebung umfasst: Erfassen von Farbbildinformationen der Referenzsubstanz unter der zweiten Umgebung und Durchführen einer Farbbildinformationsverarbeitung gemäß einem voreingestellten Bildalgorithmus; Durchführen einer Chromazitätsextraktion an den verarbeiteten Farbbildinformationen der Referenzsubstanz unter der zweiten Umgebung, um eine vierte Chromazität der Referenzsubstanz unter der zweiten Umgebung zu erhalten; Erfassen von Farbbildinformationen der Referenzsubstanz unter einer ersten Umgebung und Durchführen einer Farbbildinformationsverarbeitung gemäß einem voreingestellten Bildalgorithmus; Durchführen einer Chromazitätsextraktion an den verarbeiteten Farbbildinformationen der Referenzsubstanz unter der ersten Umgebung, um eine dritte Chromazität der Referenzsubstanz unter der ersten Umgebung zu erhalten, vorzugsweise, wobei der voreingestellte Bildalgorithmus mindestens Folgendes aufweist: Bildtransformation, Auswahl von Schlüsselbereichen, Kantentest, Rauschunterdrückung, Glättung und Verbesserung der Chromazität, vorzugsweise, wobei die Farbbildinformationen-Chromazitätsextraktion der Farbbildinformationen unter der zweiten Umgebung und die Farbbildinformationen-Chromazitätsextraktion unter der ersten Umgebung unter einem gleichen Farbauszugsystem durchgeführt werden und das Farbauszugsystem eines der folgenden ist: RGB, HSV, CMYK, CIE und LAB.

4. Das Wasserqualitätstestverfahren gemäß Anspruch 1, **dadurch gekennzeichnet, dass** bei der Erstellung des Chromazitätskorrekturmodells die Erstellung des Chromazitätskorrekturmodells unter Verwendung eines Neuronalnetzwerk-Algorithmus und eines Multivariaten nichtlinearen Fitting-Verfahrens durchgeführt wird und die beiden erstellten Chromazitätskorrekturmodelle jeweils auf der Grundlage einer bestimmten ausgewählten Referenzsubstanz trainiert werden und Trainingsergebnisse der beiden Chromazitätskorrekturmodelle erhalten werden; wobei die Trainingsergebnisse der beiden Chromazitätskorrekturmodelle verglichen werden: wenn der Absolutwert einer Differenz zwischen den beiden kleiner als ein vordefinierter Schwellenwert ist, wird optional ein Chromazitätskorrekturmodell als endgültiges Chromazitätskorrekturmodell ausgewählt; wenn der Absolutwert der Differenz zwischen den beiden größer oder gleich einem vordefinierten Schwellenwert ist, werden die beiden jeweils mit einer Standardchromazität der ausgewählten Referenzsubstanz verglichen, und ein Chromazitätskorrekturmodell, das einem Trainingsergebnis mit einem kleineren Absolutwert der Differenz zur Standardchromazität der ausgewählten Referenzsubstanz entspricht, wird als endgültiges Chromazitätskorrekturmodell ausgewählt.

5. Das Wasserqualitätstestverfahren gemäß Anspruch 3.
**dadurch gekennzeichnet, dass** vor der Durchführung der Farbbildinformationsverarbeitung an den Farbbildinformationen der Referenzsubstanz gemäß dem voreingestellten Bildalgorithmus das Verfahren ferner aufweist: Feststellen, dass die Farbbildinformationen der Referenzsubstanz eine Prüfanforderung erfüllen, und Feststellen, dass die Farbbildinformationen der Testprobe die Prüfanforderung erfüllen, wobei die Prüfanforderung vorzugsweise mindestens Folgendes aufweist: Bildpixel, Beleuchtungsintensität und Lichtgleichmäßigkeit eines Farbbildes.

6. Das Wasserqualitätstestverfahren gemäß Anspruch 1,
**dadurch gekennzeichnet, dass** das Ermitteln einer Farbwert-Chromazitäts-Zuordnungsbeziehung umfasst: Konstruieren der Farbwert-Chromazitäts-Zuordnungsbeziehung, aufweisend: Auswählen einer Vielzahl von wässrigen Lösungen, die die Zieltestsubstanz enthalten, wobei die Konzentration der Zieltestsubstanz in jeder wässrigen Lösung bekannt und unterschiedlich ist; Erfassen der Chromazitäten der Vielzahl von wässrigen Lösungen, die die Zieltestsubstanz enthalten, unter der zweiten Umgebung; Ermitteln einer Funktionsbeziehung zwischen der Konzentration der Zieltestsubstanz und der Chromazität der wässrigen Lösung der Zieltestsubstanz unter der zweiten Umgebung auf der Grundlage der Konzentration der Zieltestsubstanz in jeder wässrigen Lösung und der entsprechenden Chromazität jeder wässrigen Lösung unter der zweiten Umgebung und Ermitteln der Chromazitätskonzentrations-Zuordnungsbeziehung aus der Funktionsbeziehung.

7. Das Verfahren nach Anspruch 1, **dadurch gekennzeichnet, dass** die Referenzsubstanz eine Standardfarbkarte ist; auf der Standardfarbkarte mehrere Farbbereiche vorhanden sind, wobei bei der Durchführung einer Referenz-Stofffarbgrad-Extraktion der Chromazität der Referenzsubstanz die Chromazitäten aller Farbbereiche auf der Standardfarbkarte gleichzeitig extrahiert werden, um einen ersten Chromazitätssatz zu erhalten; wobei der erste Chromazitätssatz als Trainingsprobe einer Erstellung eines Chromazitätskorrekturmodells unterzogen wird, oder die Referenzsubstanz ein Papier-Mikrofluidik-Chip, eine Farbkarte, ein Mikrokanal-Chip oder ein Farbentwicklungsgerät ist; wobei die Referenzsubstanz durch einen der folgenden Modi gefärbt wird: eine intrinsische Farbe der Referenzsubstanz, Zugabe von Farbstoff, Zugabe eines Farbreaktionssystems.

8. Wasserqualitätsprüfgerät zur Verwendung mit einer Referenzsubstanz, umfassend:

ein Mischmodul, das dazu ausgebildet ist, eine Testprobe zu erhalten, wobei die Testprobe eine Zieltestsubstanz aufweist;
ein Erfassungsmodul, das dazu ausgebildet ist, eine zu der Testprobe korrespondierende Referenzsubstanz zu bestimmen und eine erste Chromazität der Testprobe unter der ersten Umgebung, eine dritte Chromazität der Referenzsubstanz unter einer ersten Umgebung und eine vierte Chromazität der Referenzsubstanz unter einer zweiten Umgebung zu erfassen;
ein Korrekturmodell-Konstruktionsmodul, das dazu ausgebildet ist, dass es:

einen Absolutwert der Chromazitätsdifferenz zwischen der vierten Chromazität und der dritten Chromazität zu bestimmen,
den ermittelten Absolutwert der Chromazitätsdifferenz mit einem voreingestellten Schwellenwert zu vergleichen und
wenn der Absolutwert der Chromazitätsdifferenz größer als ein vordefinierter Schwellenwert ist, ein Chromazitätskorrekturmodell durch einen Neuronalnetzwerk-Algorithmus oder ein Multivariates nichtlineares Fitting-Verfahren entsprechend einer Chromazitätsdifferenz zwischen der vierten Chromazität und der dritten Chromazität zu erstellen,
ein Korrekturmodul, das dazu ausgebildet ist, die zweite Chromazität der Testprobe unter einer zweiten Umgebung auf der Grundlage der ersten Chromazität und des vom Korrekturmodell-Konstruktionsmodul konstruierten Chromazitätskorrekturmodells zu bestimmen; und

ein Modul zur Bestimmung der Konzentration der Zieltestsubstanz, das dazu ausgebildet ist, eine Konzentration der Zieltestsubstanz auf der Grundlage einer voreingestellten Chromazitätskonzentration und auf der Grundlage der vom Korrekturmodul bestimmten zweiten Chromazität zu bestimmen,

wobei in einem Fall, in dem der Absolutwert der Chromazitätsdifferenz zwischen der vierten Chromazität und der dritten Chromazität als kleiner oder gleich dem voreingestellten Schwellenwert für Abweichungen bestimmt wird,

wobei das Modul zur Bestimmung der Konzentration der Zieltestsubstanz ferner dazu ausgebildet ist, die Konzentration der Zieltestsubstanz in der Testprobe auf der Grundlage der voreingestellten Chromazitätskonzentrations-Zuordnungsbeziehung und der Chromazität der Testprobe unter der ersten Umgebung zu ermitteln,

wobei die voreingestellte Chromazitätskonzentrations-Zuordnungsbeziehung eine Zuordnungsbeziehung zwischen der Chromazität der Testprobe unter der zweiten Umgebung und der Konzentration der Zieltestsubstanz widerspiegelt, und

wobei die erste Umgebung eine Testortumgebung ist, in der sich die Testprobe befindet; und die zweite Umgebung eine Laborstandardumgebung ist.

9. Das Wasserqualitätsprüfgerät nach Anspruch 8, wobei das Mischmodul dazu ausgebildet ist, zu beurteilen, ob eine wässrige Lösung der Zieltestsubstanz gefärbt ist oder nicht und ob die wässrige Lösung der Zieltestsubstanz eine Interferenzfarbe oder eine heiße Farbe aufweist, so dass bei der Verwendung die wässrige Lösung der Zieltestsubstanz als Testprobe genommen wird, wenn die wässrige Lösung der Zieltestsubstanz gefärbt ist und keine Interferenzfarbe aufweist; oder ein entsprechendes Testreagenz entsprechend der Zieltestsubstanz ausgewählt wird, wenn die wässrige Lösung der Zieltestsubstanz farblos ist oder eine Interferenzfarbe aufweist, und das ausgewählte Testreagenz mit der wässrigen Lösung der Zieltestsubstanz verschmolzen wird, um eine Testprobe zu erhalten.

10. Das Wasserqualitätsprüfgerät gemäß Anspruch 8, wobei zum Erfassen der dritten Chromazität der Referenzsubstanz unter der ersten Umgebung und der vierten Chromazität der Referenzsubstanz unter der zweiten Umgebung das Erfassungsmodul dazu ausgebildet ist, Farbbildinformationen der Referenzsubstanz unter der zweiten Umgebung zu erfassen und eine Farbbildinformationsverarbeitung gemäß einem voreingestellten Bildalgorithmus durchzuführen; um eine Chromazitätsextraktion an den verarbeiteten Farbbildinformationen der Referenzsubstanz unter der zweiten Umgebung durchzuführen, um die vierte Chromazität der Referenzsubstanz unter der zweiten Umgebung zu erhalten; um Farbbildinformationen der Referenzsubstanz unter der ersten Umgebung zu erfassen und eine Farbbildinformationsverarbeitung gemäß einem voreingestellten Bildalgorithmus durchzuführen; eine Farbbildinformationen-Chromazitätsextraktion an den verarbeiteten Farbbildinformationen der Referenzsubstanz unter der ersten Umgebung durchzuführen, um die dritte Chromazität der Referenzsubstanz unter der ersten Umgebung zu erhalten, wobei der voreingestellte Bildalgorithmus vorzugsweise mindestens Folgendes aufweist: Bildtransformation, Auswahl von Schlüsselbereichen, Kantentest, Rauschunterdrückung, Glättung und Verbesserung der Chromazität.

11. Das Wasserqualitätsprüfgerät nach Anspruch 10,

dadurch gekennzeichnet, dass das Erfassungsmodul ferner dazu ausgebildet ist, zu bestimmen, ob die Farbbildinformationen der Referenzsubstanz eine Prüfanforderung erfüllen, und zu bestimmen, ob die Farbbildinformationen der Testprobe die Prüfanforderung erfüllen; in einem Fall, in dem festgestellt wird, dass die Farbbildinformationen der Referenzsubstanz die Prüfanforderung erfüllen und die Farbbildinformationen der Testprobe die Prüfanforderung erfüllen, eine Farbbildinformationsverarbeitung an den Farbbildinformationen der Referenzsubstanz und an den Farbbildinformationen der Testprobe gemäß einem voreingestellten Bildalgorithmus durchgeführt wird,

vorzugsweise weist das Erfassungsmodul folgende Komponenten auf: ein Bildaufnahmemodul, das dazu ausgebildet ist, Farbbildinformationen der Referenzsubstanz unter einer zweiten Umgebung zu erfassen und Farbbildinformationen der Referenzsubstanz und Farbbildinformationen der Zieltestsubstanz unter einer ersten Umgebung zu erfassen; ein Bildverarbeitungsmodul, das dazu ausgebildet ist, eine Farbbildinformationsverarbeitung an den Farbbildinformationen der Referenzsubstanz und den Farbbildinformationen der Zieltestsubstanz gemäß einem vordefinierten Bildalgorithmus durchzuführen und eine Farbbildinformationen-Chromazitätsextraktion an den verarbeiteten Farbbildinformationen durchzuführen, vorzugsweise weist das Bildaufnahmemodul eine Kamera, ein Mobiltelefon, eine Kamera, einen Scanner oder ein Überwachungsgerät auf.

12. Computerlesbares Speichermedium, **dadurch gekennzeichnet, dass** auf dem computerlesbaren Speichermedium Befehle gespeichert sind, die, wenn sie auf dem Gerät gemäß einem der Ansprüche 8 bis 11 ausgeführt werden, das Gerät veranlassen, das Wasserqualitätstestverfahren gemäß einem der Ansprüche 1 bis 7 durchzuführen.

**Revendications**

1. Procédé d'essai de la qualité de l'eau comprenant : l'obtention d'un échantillon d'essai, l'échantillon d'essai comprenant une substance d'essai cible ; la détermination d'une substance de référence correspondant à l'échantillon d'essai ; la détermination d'une troisième chromaticité de la substance de référence dans un premier environnement, et d'une quatrième chromaticité de la substance de référence dans un second environnement ;

l'obtention d'une relation de correspondance chromaticité-concentration caractérisant une relation de correspondance entre la chromaticité de l'échantillon d'essai dans le second environnement et la concentration de la substance d'essai cible ;
la détermination d'une valeur absolue de la différence de chromaticité entre la quatrième chromaticité et la troisième chromaticité, et
o si une valeur absolue de la différence de chromaticité entre la quatrième chromaticité et la troisième chromaticité est supérieure à un seuil d'écart prédéfini : la construction d'un modèle de correction de la chromaticité par un algorithme de réseau neuronal ou un procédé d'ajustement non linéaire à plusieurs variables en fonction d'une différence de chromaticité entre la quatrième chromaticité et la troisième chromaticité ;
l'acquisition d'une première chromaticité de l'échantillon d'essai dans le premier environnement ; la détermination d'une deuxième chromaticité de l'échantillon d'essai dans le second environnement sur la base de la première chromaticité et du modèle de correction de la chromaticité ; et la détermination d'une concentration de la substance d'essai cible sur la base de la deuxième chromaticité déterminée et de la relation de correspondance chromaticité-concentration ; et ∘ dans le cas où la valeur absolue de la différence de chromaticité entre la quatrième chromaticité et la troisième chromaticité est inférieure ou égale au seuil d'écart préétabli,

le procédé comprend en outre : l'acquisition de la chromaticité de l'échantillon d'essai dans le premier environnement ; et
l'obtention de la concentration de la substance d'essai cible dans l'échantillon d'essai sur la base de la relation chromaticité-concentration et de la chromaticité de l'échantillon d'essai dans le premier environnement,
dans lequel le premier environnement est un environnement de site d'essai dans lequel se trouve l'échantillon d'essai ; et le second environnement est un environnement standard de laboratoire.

2. Procédé d'essai de la qualité de l'eau selon la revendication 1, **caractérisé en ce que** l'obtention de l'échantillon d'essai comprend : le jugement si une solution aqueuse de la substance d'essai cible est colorée ou non, et le jugement si la solution aqueuse de la substance d'essai cible a une couleur d'interférence ou non ; la prise de la solution aqueuse de la substance d'essai cible comme échantillon d'essai si la solution aqueuse de la substance d'essai cible est colorée et n'a pas de couleur d'interférence ; la sélection d'un réactif d'essai correspondant en fonction de la substance d'essai cible si la solution aqueuse de la substance d'essai cible est incolore ou a une couleur d'interférence, et la fusion du réactif d'essai sélectionné avec la solution aqueuse de la substance d'essai cible afin d'obtenir un échantillon d'essai,
de préférence, dans lequel le réactif d'essai correspondant à la substance d'essai cible est un réactif d'essai qui subit une réaction chromogène lors de sa fusion avec une solution aqueuse de la substance d'essai cible.

3. Procédé d'essai de la qualité de l'eau selon la revendication 1, **caractérisé en ce que** la détermination de la troisième chromaticité de la substance de référence dans le premier environnement et de la quatrième chromaticité de la substance de référence dans le second environnement comprend : l'acquisition d'informations d'image couleur de la substance de référence dans le second environnement, et la réalisation d'un traitement d'informations d'image couleur selon un algorithme d'image prédéfini ; la réalisation d'une extraction de chromaticité sur les informations d'image couleur traitées de la substance de référence dans le second environnement pour obtenir une quatrième chromaticité de la substance de référence dans le second environnement ; l'acquisition d'informations d'image couleur de la substance de référence dans un premier environnement, et la réalisation d'un traitement d'informations d'image couleur selon un algorithme d'image prédéfini ; la réalisation d'une extraction de chromaticité sur les informations d'image couleur traitées de la substance de référence dans le premier environnement pour obtenir une troisième chromaticité de la substance de référence dans le premier environnement,

de préférence, l'algorithme d'image prédéfini comprend au moins : la transformation de l'image, la sélection des régions clés, le contrôle des bords, la réduction du bruit, le lissage et l'amélioration de la chromaticité,

de préférence, l'extraction de la chromaticité des informations d'image couleur dans le second environnement et l'extraction de la chromaticité des informations d'image couleur dans le premier environnement sont effectuées dans le cadre d'un même système de chromaticité, et le système de chromaticité est l'un quelconque des systèmes suivants : RGB, HSV, CMYK, CIE et LAB.

4. Procédé d'essai de la qualité de l'eau selon la revendication 1, **caractérisé en ce que** lorsque la construction du modèle de correction de la chromaticité est effectuée, la construction du modèle de correction de la chromaticité est effectuée en utilisant un algorithme de réseau neuronal et un procédé d'ajustement non linéaire à plusieurs variables respectivement, et les deux modèles de correction de la chromaticité construits sont entraînés respectivement sur la base d'une certaine substance de référence sélectionnée, et les résultats d'entraînement des deux modèles de correction de la chromaticité sont obtenus ; les résultats d'entraînement des deux modèles de correction de la chromaticité sont comparés : lorsque la valeur absolue d'une différence entre les deux est inférieure à un seuil prédéfini, un modèle de correction de la chromaticité est éventuellement sélectionné comme modèle de correction de la chromaticité final ; lorsque la valeur absolue d'une différence entre les deux est supérieure ou égale à un seuil prédéfini, les deux sont comparés à une chromaticité standard de la substance de référence sélectionnée, respectivement, et un modèle de correction de la chromaticité correspondant à un résultat d'entraînement présentant une plus petite valeur absolue d'une différence avec la chromaticité standard de la substance de référence sélectionnée est sélectionné comme modèle de correction de la chromaticité final.

5. Procédé d'essai de la qualité de l'eau selon la revendication 3, **caractérisé en ce qu'**avant d'effectuer le traitement des informations d'image couleur sur les informations d'image couleur de la substance de référence selon l'algorithme d'image prédéfini, le procédé comprend en outre : la détermination que les informations d'image couleur de la substance de référence répond à une exigence d'essai, et la détermination que les informations d'image couleur de l'échantillon d'essai répond à l'exigence d'essai,

de préférence, dans lequel l'exigence d'essai comprend au moins : les pixels d'image, l'intensité d'éclairage et l'uniformité de lumière d'une image en couleur.

6. Procédé d'essai de la qualité de l'eau selon la revendication 1, **caractérisé en ce que** l'obtention d'une relation de cartographie chromaticité-concentration comprend : la construction de la relation de cartographie chromaticité-concentration comprenant : la sélection d'une pluralité de solutions aqueuses contenant la substance d'essai cible, la concentration de la substance d'essai cible dans chaque solution aqueuse étant connue et différente ; l'acquisition des chromaticités de la pluralité de solutions aqueuses contenant la substance d'essai cible dans le second environnement ; l'obtention d'une relation fonctionnelle entre la concentration de la substance d'essai cible et la chromaticité de la solution aqueuse de la substance d'essai cible dans le second environnement, sur la base de la concentration de la substance d'essai cible dans chaque solution aqueuse et de la chromaticité correspondante de chaque solution aqueuse dans le second environnement, et l'obtention de la relation de correspondance chromaticité-concentration à partir de la relation fonctionnelle.

7. Procédé selon la revendication 1, **caractérisé en ce que** la substance de référence est une carte de couleur standard ; une pluralité de régions de couleur existent sur la carte de couleur standard, lors de l'extraction du degré de couleur de la substance de référence, les chromaticités de toutes les régions de couleur sur la carte de couleur standard sont extraites simultanément pour obtenir un premier ensemble de chromaticité ; le premier ensemble de chromaticité est soumis à la construction d'un modèle de correction de chromaticité en tant qu'échantillon d'entraînement, ou

la substance de référence est une puce microfluidique en papier, une carte couleur, une puce à microcanaux ou un appareil de développement des couleurs ; la substance de référence est colorée selon l'un quelconque des modes suivants : une couleur intrinsèque de la substance de référence, un ajout d'un colorant, un ajout d'un système de réaction colorée.

8. Dispositif d'essai de la qualité de l'eau à utiliser avec une substance de référence, comprenant : un module de mélange configuré pour obtenir un échantillon d'essai, l'échantillon d'essai comprenant une substance d'essai cible ; un module d'acquisition configuré pour déterminer une substance de référence correspondant à l'échantillon d'essai, et acquérir une première chromaticité de l'échantillon d'essai dans le premier environnement, une troisième chromaticité de la substance de référence dans un premier environnement, et une quatrième chromaticité de la substance de référence dans un second environnement ;

un module de construction de modèle de correction configuré pour

déterminer une valeur absolue de la différence de chromaticité entre la quatrième chromaticité et la troisième chromaticité, comparer la valeur absolue déterminée de la différence de chromaticité à un seuil d'écart prédéfini, et si la valeur absolue de la différence de chromaticité est supérieure à un seuil d'écart prédéfini, construire un modèle de correction de la chromaticité par un algorithme de réseau neuronal ou un procédé d'ajustement non linéaire à plusieurs variables en fonction d'une différence de chromaticité entre la quatrième chromaticité et la troisième chromaticité, un module de révision configuré pour déterminer la deuxième chromaticité de l'échantillon d'essai dans un second environnement sur la base de la première chromaticité et du modèle de correction de la chromaticité construit par le module de construction du modèle de correction ; et un module de détermination de la concentration de la substance d'essai cible configuré pour déterminer une concentration de la substance d'essai cible sur la base d'une relation de correspondance chromaticité-concentration prédéfinie et de la deuxième chromaticité déterminée par le module de révision, dans le cas où la valeur absolue de la différence de chromaticité entre la quatrième chromaticité et la troisième chromaticité est inférieure ou égale au seuil d'écart prédéfini, le module de détermination de la concentration de la substance d'essai cible est en outre configuré pour obtenir la concentration de la substance d'essai cible dans l'échantillon d'essai sur la base de la relation de correspondance chromaticité-concentration prédéfinie et de la chromaticité de l'échantillon d'essai dans le premier environnement, la relation de correspondance chromaticité-concentration prédéfinie reflétant une relation de correspondance entre la chromaticité de l'échantillon d'essai dans le second environnement et la concentration de la substance d'essai cible, et

dans lequel le premier environnement est un environnement de site d'essai dans lequel se trouve l'échantillon d'essai ; et le second environnement est un environnement standard de laboratoire.

9. Dispositif d'essai de la qualité de l'eau selon la revendication 8, dans lequel le module de mélange est configuré pour juger si une solution aqueuse de la substance d'essai cible est colorée ou non, et si la solution aqueuse de la substance d'essai cible a une couleur d'interférence ou non, de sorte que, lors de l'utilisation, la solution aqueuse de la substance d'essai cible est prise comme échantillon d'essai si la solution aqueuse de la substance d'essai cible est colorée et n'a pas de couleur d'interférence ; soit un réactif d'essai correspondant à la substance d'essai cible est sélectionné si la solution aqueuse de la substance d'essai cible est incolore ou présente une couleur d'interférence, et le réactif d'essai sélectionné est fusionné avec la solution aqueuse de la substance d'essai cible pour obtenir un échantillon d'essai.

10. Dispositif d'essai de la qualité de l'eau selon la revendication 8, dans lequel, pour acquérir la troisième chromaticité de la substance de référence dans le premier environnement et la quatrième chromaticité de la substance de référence dans le second environnement, le module d'acquisition est configuré pour acquérir des informations d'image couleur de la substance de référence dans le second environnement, et pour effectuer le

traitement des informations d'image couleur selon un algorithme d'image prédéfini ; l'extraction de chromaticité sur les informations d'image couleur traitées de la substance de référence dans le second environnement pour obtenir la quatrième chromaticité de la substance de référence dans le second environnement ; pour acquérir des informations d'image couleur de la substance de référence dans le premier environnement, et la réalisation du traitement des informations d'image couleur selon un algorithme d'image prédéfini ; la réalisation de l'extraction de chromaticité sur les informations d'image couleur traitées de la substance de référence dans le premier environnement pour obtenir la troisième chromaticité de la substance de référence dans le premier environnement, de préférence, l'algorithme d'image prédéfini comprend au moins :

la transformation de l'image, la sélection des régions clés, le test en périphérie, la réduction du bruit, le lissage et l'amélioration de la chromaticité.

11. Dispositif d'essai de la qualité de l'eau selon la revendication 10, **caractérisé en ce que** le module d'acquisition est en outre configuré pour déterminer si les informations d'image couleur de la substance de référence répondent à une exigence d'essai, et déterminer si les informations d'image couleur de l'échantillon d'essai répondent à l'exigence d'essai ; dans le cas où il est déterminé que les informations d'image couleur de la substance de référence répondent à l'exigence d'essai, et que les informations d'image couleur de l'échantillon d'essai répondent à l'exigence d'essai, le traitement des informations d'image couleur est effectué sur les informations d'image couleur de la substance de référence et sur les informations d'image couleur de l'échantillon d'essai selon un algorithme d'image prédéfini,

de préférence, le module d'acquisition comprend : un module d'acquisition d'images, configuré pour acquérir des informations d'images couleur de la substance de référence dans un second environnement ; et acquérir des informations d'images couleur de la substance de référence et des informations d'images couleur de la substance d'essai cible dans un premier environnement ; un module de traitement d'images, configuré pour effectuer un traitement des informations d'images couleur sur les informations d'images couleur de la substance de référence et les informations d'images couleur de la substance d'essai cible selon un algorithme d'image prédéfini, et effectuer une extraction de chromaticité sur les informations d'images couleur traitées,

de préférence, le module d'acquisition d'images comprend un appareil photo, un téléphone portable, une caméra, un scanner ou un appareil de surveillance.

12. Support de stockage lisible par ordinateur, **caractérisé en ce que** le support de stockage lisible par ordinateur contient des instructions qui, lorsqu'elles sont exécutées sur le dispositif selon l'une quelconque des revendications 8 à 11, permettent au dispositif d'exécuter le procédé d'essai de la qualité de l'eau selon l'une quelconque des revendications 1 à 7.

obtaining a test sample, the test sample including a target test substance — S101

↓

determining a reference substance corresponding to the test sample — S102

↓

constructing a chromaticity correction model based on the reference substance — S103

↓

acquiring a first chromaticity of the test sample under a first environment — S104

↓

determining a second chromaticity of the test sample under a second environment based on the first chromaticity and the chromaticity correction model — S105

↓

determining a concentration of the target test substance based on the second chromaticity — S106

FIG. 1

mixing module — acquisition module — correction model construction module — revision module — target test substance concentration determining module

FIG. 2

FIG. 3

FIG. 4

FIG. 5

FIG. 6

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Estimation of sulfonamides concentration in water based on digital colourimetry. **CARVALHO, PEDRO H. et al.** Iberian Conference on Pattern Recognition and Image Analysis.. Springer International Publishing, 2019, 355-366

- **LOPEZ-RUIZ NURIA et al.** Smartphone-based simultaneous pH and nitrite colorimetric determination for paper microfluidic devices. *Analytical chemistry*, 2014, vol. 86 (19), 9554-9562